# EUROPEAN PATENT SPECIFICATION

(11) **EP 1 633 417 B1**
(45) Date of publication and mention of the grant of the patent: **31.01.2007**
(21) Application number: 04751762.8
(22) Date of filing: 25.05.2004
(51) Int. Cl.: A61M 5/19

(54) **MULTIPLE CHAMBER MEDICATION DISPENSING APPARATUS**
MEHRKAMMER MEDIKAMENTENVERABREICHUNGSVORRICHTUNG
APPAREIL DE DISTRIBUTION DE MEDICAMENT A CHAMBRES MULTIPLES

(30) Priority: 30.05.2003 US 474787 P
(43) Date of publication of application: 15.03.2006
(73) Proprietor: ELI LILLY AND COMPANY, Indianapolis, Indiana 46285 (US)
(72) Inventor: DAI, He, Carmel, Indiana 46033 (US); MORRISON, William, Harold, Junior, Avon, Indiana 46123 (US); NESBITT, Robert, Richardson, Fishers, Indiana 46038 (US); SCHAFF, Anthony, Lawrence, Senior, Carmel, Indiana 46033 (US)
(74) Representative: Ingham, Stephen H.
(86) International application number: PCT/US2004/014532
(87) International publication number: WO 2004/108193

(56) References cited:
- GB-A- 2 139 708
- US-A- 4 583 978
- US-A- 5 078 691
- US-A- 5 378 233
- US-A1- 2002 068 907

## Description

### BACKGROUND OF THE INVENTION

The present invention pertains to medication dispensing devices, and, in particular, to a portable medication dispensing device such as an injector pen.

Patients suffering from a number of different diseases frequently must inject themselves with medication. To allow a person to conveniently and accurately self-administer medicine, a variety of devices broadly known as injector pens or injection pens have been developed. Generally, these pens are equipped with a cartridge including a piston and containing a multi-dose quantity of liquid medication. A drive member, extending from within a base of the injector pen and operably connected with typically more rearward mechanisms of the pen that control drive member motion, is movable forward to advance the piston in the cartridge in such a manner to dispense the contained medication from an outlet at the opposite cartridge end, typically through a needle that penetrates a stopper at that opposite end. In disposable or prefilled pens, after a pen has been utilized to exhaust the supply of medication within the cartridge, the entire pen is discarded by a user, who then begins using a new replacement pen. In reusable pens, after a pen has been utilized to exhaust the supply of medication within the cartridge, the pen is disassembled to allow replacement of the spent cartridge with a fresh cartridge, and then the pen is reassembled for it subsequent use.

While suitable to deliver useful therapeutics, a variety of available injector pens are not without their shortcomings. One shortcoming of some injector pens is that the plunging force required to advance the cartridge piston during an injection may be too large for some users, such as those with limited hand strength, possibly due to the disease being treated. Another shortcoming of some injector pens is that the drive member motion results in a dose accuracy that may be insufficient for some applications. And, while for a given drive member motion, a smaller diameter conventional cartridge in a pen generally can be used to obtain higher dose accuracy, such a smaller diameter cartridge either limits the amount of medicine contained in the entire cartridge, or requires a longer cartridge length, which may be unacceptable to some pen users.

One known syringe disclosed in U.S. Patent Re. 32,974 utilizes a smaller diameter dose chamber and a larger diameter storage chamber within the syringe housing, each chamber having its associated piston. While the overall syringe design may allow an injection to be performed with lower plunging or glide forces, as the syringe teaches the injection needle not be mounted during part of the time the syringe is being manipulated, the syringe operation is potentially confusing and complicated for users familiar with the operations of more conventional injector pen technology.

Thus, it would be desirable to provide an apparatus that overcomes one or more of these and other shortcomings of the prior art.

### BRIEF SUMMARY OF THE INVENTION

In one form thereof, the present invention provides a medication dispensing apparatus including a fluid container, first and second plungers, a drive assembly, and an actuator. The fluid container defines a medication-filled reservoir including an injection chamber and a storage chamber in fluid flow communication. The first plunger includes a piston sealing an end of the injection chamber. The first plunger is movable relative to the fluid container while maintaining the seal to alter the volume of the injection chamber. The second plunger is movable relative to the fluid container while maintaining the seal to alter the volume of the storage chamber. The sealed end of the injection chamber has a cross-sectional area that is smaller than a cross-sectional area of the sealed end of the storage chamber. The drive assembly is drivingly connected to the first plunger and the second plunger. The drive assembly is operable during dose setting to shift the first and second plungers relative to the fluid container different distances so as to reduce the volume of the storage chamber while simultaneously increasing the volume of the injection chamber a similar amount. The actuator is movable to shift the first plunger relative to the fluid container to reduce the volume of the injection chamber to force medicine in the reservoir through an outlet of the fluid container.

One advantage of the present invention is that a medication dispensing apparatus can be provided with a small area injection chamber that permits injections to be performed with a low glide force, which low glide force can allow for a direct injection as opposed to a mechanically assisted injection.

Another advantage of the present invention is that a medication dispensing apparatus can be provided which uses multiple chambers, but which outwardly appears to operate similar to some conventional injector pens.

Another advantage of the present invention is that a medication dispensing apparatus can be provided with high dose accuracy, and which can accurately administer small volume doses, thereby being useful for high potency drugs.

Still another advantage of the present invention is that a medication dispensing apparatus can be provided with an actuator that can be distinct from the dose setting element, which actuator form may be familiar to people who use conventional syringes.

Still another advantage of the present invention is that a medication dispensing apparatus can be provided which is designed to contain a number of different volumes in its chambers, and which chambers can be a variety of shapes, thereby allowing a small overall device design that is well shaped for discrete usage.

Yet another advantage of the present invention is that a medication dispensing apparatus can be provided that is manufacturable with any one of a number of gear ratios to allow an appropriate actuator travel for expected injections, such as a gear ratio to achieve a sufficiently large travel of the actuator even for small volume doses.

Yet another advantage of the present invention is that a medication dispensing apparatus can provided with a variety of storage chambers to allow ready mixing of materials or delivery of any one of various therapeutics within the chambers.

### BRIEF DESCRIPTION OF THE DRAWINGS

The above-mentioned and other advantages and objects of this invention, and the manner of attaining them, will become more apparent, and the invention itself will be better understood by reference to the following description of embodiments of the invention taking in conjunction with the accompanying drawings, wherein:
Fig. 1 is a diagrammatic perspective view of a first embodiment of a medication dispensing apparatus with dual chambers of the present invention;
Fig. 2 is a diagrammatic cross-sectional view of select portions of the medication dispensing apparatus of Fig. 1 in a ready-to-be-dosed state;
Fig. 3 is a diagrammatic perspective view of the portions of Fig. 2, but after the apparatus has been manipulated from its ready-to-be-dosed state to a ready-to-inject state;
Fig. 4 is a diagrammatic cross-sectional view of a one-way valve that assists in controlling the medicine reservoir outlet during use;
Fig. 5A is a diagrammatic front view of select portions of another embodiment of the medication dispensing apparatus of the present invention during dose setting; and.
Fig. 5B is a diagrammatic front view of the portions of Fig. 5A, but during dose injecting.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

### DETAILED DESCRIPTION OF THE INVENTION

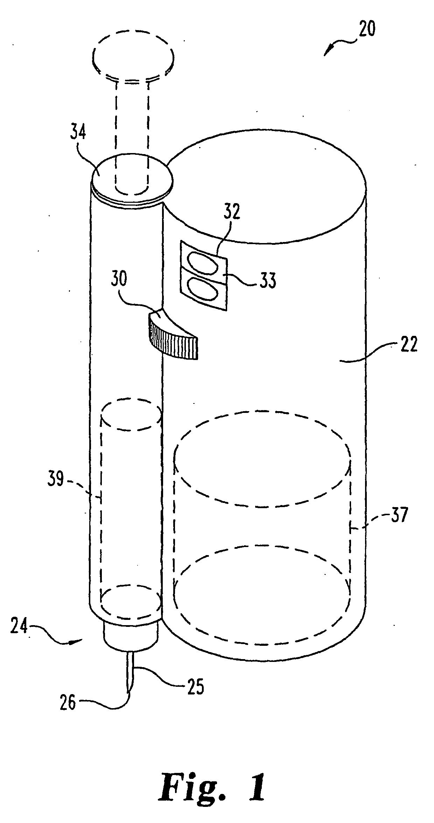

Referring now to Fig. 1, there is shown a first embodiment of a medication dispensing apparatus with multiple chambers of the present invention. The apparatus, generally designated 20, is an injector pen that is portable by a user to discreetly administer, at any appropriate time and place, a dose of medication selected by the user. Injector pen 20 is shorter and wider in overall shape than some more conventionally shaped injector pens, but such shape results in a desirably compact design, and further can be modified within the scope of the invention.

Medication injector pen 20 is a disposable or prefilled pen, in that after the quantity of medicine contained therein is exhausted by multiple operations of the pen, the entire pen is discarded rather than being reset and reloaded with a replacement container of medicine. As the pen has a relatively short working life, the pen components are preferably molded from one or more polymeric materials such as plastic when possible for cost effectiveness. Other more robust and/or more durable materials of construction may also be used, especially in reusable pens of the present invention which have a longer expected life.

Injector pen 20 includes an external housing 22 that is shown as having an exterior with different sized lobes, which lobes appear as if formed by interconnected, different diameter cylinders. This housing shape, which relates to cylindrical aspects of the fluid container described below, may be changed in alternate embodiments to other shapes, such as more ergonomic or aesthetic shapes, as well as a more box-like shape. Housing 22 has a distal end to which is removably mounted a needle assembly 24 including a double-ended needle cannula or injection needle 25 having a distal tip 26 at one end and a not-shown proximal point at the other. Although the needle assembly is shown as having a single injection needle, needle assemblies which may be used with the present invention may be of various types known in the art, including, but not limited to, assemblies with one or more shortened injection needles, including microneedle arrays.

A knurled wheel 30 is rotatably mounted on housing 22 and manually rotatable by the user to set a dose for delivery. The positioning of wheel 30 at the intersection of the housing lobes improves user access to the wheel. Wheel 30 is operably connected to a mechanism within housing 22 that visibly displays through a lens covered, window 32 of housing 22 the dose that injector pen 20 has been set to deliver by operation of wheel 30. The pen is designed to be set by increments that are adequately small in resolution for the medication involved, such as ten or five microliters. The pen also may provide an audible click or the like upon each increment during the display increasing or decreasing. The dose display is shown as a two-digit odometer type display 33 using a ones disc and a tens disc such as with the incrementing resolution being ten microliters. Other display types may be employed, such as if five microliters is the dosing increment.

Movably mounted on housing 22 is an actuator 34 used to inject medicine from the pen. Actuator 34 is shown as a plunger separate from dose setting wheel 30 and is arranged on the proximal end of the pen housing to allow it to be operated with the finger or thumb of the hand in which the housing 22 is held during dose injection. In Fig. 1, actuator 34 is shown in solid lines in its initial or zero or ready to be dosed position, at which time zero is visible on display 33. Actuator 34 is also shown in dashed lines in Fig. 1 in one of its ready to inject positions. Other actuator configurations, including a button or pivoting lever arranged on the side of the pen housing to allow it to be squeezed with one or more fingers of the hand in which the housing 22 is held, and possibly more integrated with the dose setting element, may be used in alternate embodiments.

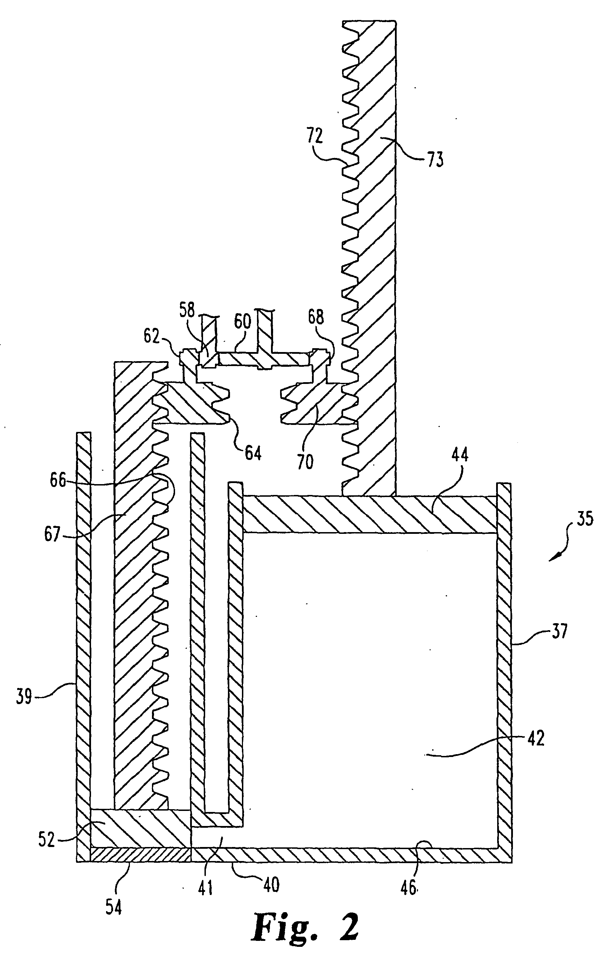

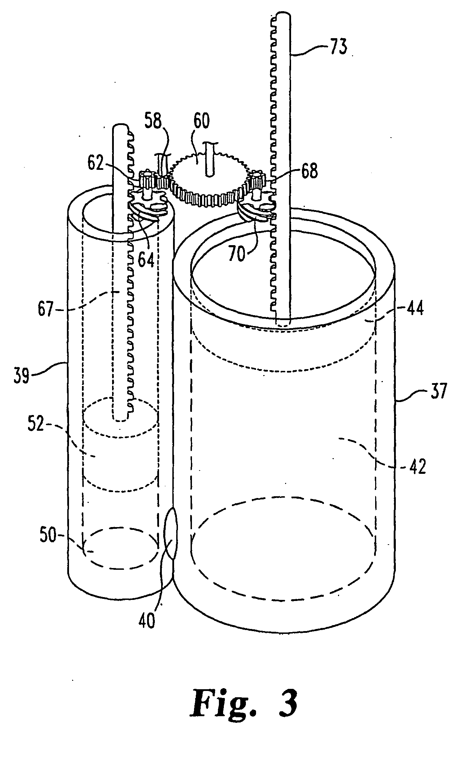

With additional reference to Figs. 2 and 3, pen 20 includes a dual chamber fluid container, generally indicated at 35, which holds a medication, such as insulin or another therapeutic. In the Figures, fluid container 35 is shown as a distinct part that is protectively housed within housing 22. In alternate embodiments, the shown housing itself can serve as the fluid container, such as by having the container aspects integrated therein. Or, the housing can include a housing portion that holds a distinct container, or which forms the container on its own, which portion can be attached to the remainder of the pen housing.

Fluid container 35 is made of a medically and mechanically suitable material, such as glass or more preferably a polymer or blend of polymer materials, such as a plastic, to facilitate its manufacture and accuracy in sizing in production. Container 35 includes a first cylindrical, tubular portion 37 and a second cylindrical, tubular portion 39 that are spanned by conduit 40. In the shown embodiment, tubular portions 37 and 39 are parallel to each other as well as arranged laterally of each other. Along their internal piston engaging lengths, tubular portion 37 has a constant inner diameter that is larger than the constant inner diameter of tubular portion 39. These constant inner diameter references relate to the fact that its associated piston maintain its seal, as naturally the tubular portion inner diameters may have slight variations, such as a draft angle if formed from plastic. While portions 37, 39 and conduit 40 are shown integrally constructed, such could be manufactured as separate pieces and then sealingly assembled together. Other cross-sectional shapes and arrangements of the tubular portions may be used in alternate embodiments.

Tubular portion 37 defines a large volume storage chamber portion 42 of the medicine-filled reservoir of fluid container 35. Storage chamber 42 is closed at its proximal end by a plunger piston 44 that is axially slidably and sealably engaged with the interior wall of tubular portion 37 to hold the fluid medication. The distal end of chamber 40 is closed by a wall 46 of the container. Wall 46 is shown having an internal surface that is flat like the distal face of piston 44, but which could be sloped up from conduit 40 to promote chamber drainage provided a corresponding modification to the piston distal face were preferably provided.

Tubular portion 39 defines a smaller volume injection chamber portion 50 of the medicine-filled reservoir of fluid container 35. Injection chamber 50 and storage chamber 42 are in flow communication via channel 41. Positioning the channel 41 such that it opens as low as possible into each of the chamber portions minimizes wastage of the contained medication at the end of the life of the pen. Plunger piston 52 closes the proximal end of injection chamber 50 and is axially slidably and sealably engaged with the interior wall of tubular portion 39. The distal, outlet end of injection chamber 50 is sealed by a septum abstractly shown at 54. In an alternate embodiment, the distal end of the tubular portion 39 can have a stepped-down neck portion shaped more similar to the distal end of a standard cartridge to better accommodate standard pen needles. In still further alternate embodiments, the cartridge and septum can be cooperatively designed to minimize waste at the end of the useful life of the cartridge or pen. For example, a polymeric cartridge can be molded with a reduced diameter pen needle mounting collar that is directly adjacent the piston containing tubular portion, rather than the collar being spaced from the tubular portion by the tapered form used in the stepped down neck portion of conventional glass cartridges. Still further and with the tubular portion distal end again having a distal end like the conventionally shaped cartridge, the septum, while allowing for penetration by the needle proximal point, can extend within the neck to fill space otherwise occupied by medicine that is wasted at the end of the pen life.

When needle assembly 24 is mounted to housing 22 as shown in Fig. 1, the proximal point of injection needle 25 penetrates septum 54 to provide a fluid flow outlet by which medicine within the fluid container reservoir can be dispensed from needle tip 26 during injection operations of injector pen 20.

Pen 20 is configured such that pistons 44 and 52 are precisely controlled during dose setting to simultaneously change the volumes of storage chamber 42 and injection chamber 50 in equal and opposite amounts, and during dose injecting to reduce the total volume of the medication reservoir in an amount equal to the dose set to be delivered. This control during injecting is preferably accomplished by reducing the volume of the injection chamber without changing the volume of the storage chamber.

This control utilizes a drive assembly including a gear set disposed between the plungers of which pistons 44 and 52 are parts. In the embodiment shown in Figs. 2 and 3, the drive assembly includes first and second intermeshing spur gears 58 and 60 of different diameters, one of which, such as spur gear 58, is rotatably driven by manual rotation of dose setting wheel 30 during dose setting. Although the spur gear can be connected with the dose setting wheel so as to have equal amounts of rotation during use, the connection may be via a gear to optimize rotational ergonomics. Because wheel 30 can be rotated as is conventional in one direction to increase a dose setting, and in the opposite direction to decrease a dose setting which is too large, but not to a setting below zero, the spur gears and the other elements of the drive assembly are journaled in the housing to rotate in either direction. Spur gear 58 intermeshes with a spur gear 62, the rotation of which gear 62 rotates a worm gear 64. Although shown in the Figures as being of a unitary construction, spur gear 62 and worm gear 64 can be formed separately and fixedly connected to rotate together. Worm gear 64 engages a longitudinally extending gear rack 66 formed on a rotatably fixed plunger shaft 67. Piston 52 is fixedly attached to plunger shaft 67 in any suitable manner to be shiftable therewith in both the proximal and distal directions relative to the fluid container 35. Similarly, spur gear 60 intermeshes with a spur gear 68 that when rotated turns a worm gear 70 that engages a longitudinally extending gear rack 72 formed on a rotatably fixed plunger shaft 73. Piston 44 is fixedly attached to plunger shaft 73 in any suitable manner to be shiftable therewith in both the proximal and distal directions relative to the fluid container 35.

The gears of the drive assembly have diameters and subsequent gear ratios selected to account for the differences in inner diameters of the injection chamber 50 and storage chamber 42. as well as relative rotation to properly affect the direction of motion of the plungers, such that during dose setting the hydraulic pressure of the medication in container 35 remains relatively constant. In this way, medication will not be discharged, nor will air be drawn in, through the needle during dose setting. In one embodiment, during dose setting, piston 52 is drivingly shifted by the drive assembly a distance which is six times as large and in the opposite direction as piston 44 is drivingly shifted by the drive assembly. In such embodiment, the inner diameter of injection chamber 50 is about 0.570 cm (0.225 inch), and the height of the tubular portion 39, which is impacted by its piston height, is large enough for the injection chamber 50 to hold a single dose in amounts up to, for example, 0.6ml, which would correspond to 60 international units for U100 insulin or 120 international units or more for U200 or even more potent or concentrated insulin solutions. And, the inner diameter of storage chamber 42 is about 1.397 cms (0.550 inch), and the height of the tubular portion 37, which is also impacted by its piston height, is large enough for the storage chamber 42 to initially hold up to, for example, 3.0mls, which amount allows the pen to be used to deliver multiple doses over its life. Different volume chambers, such as larger or smaller chambers, may be used within the scope of the invention.

The particular gear ratio that moves the injection chamber plunger a greater distance than the storage chamber plunger may be selected by the manufacturer in view of the needs of the expected users of the pen. Ratios other than the six value mentioned above may be used. Ratios smaller than six, for example two or four, can be used if the user is expected to be able to provide a greater injection force. Ratios bigger than six, for example, seven, eight, or up to twelve or more, may be used where a larger travel or throw of the actuator is desired for the target value or range to be delivered.

Actuator 34 is operably connected to plunger shaft 67. In the described embodiment, the connection may be a direct linkage that results in actuator 34 moving out from housing 22 an amount equal to the distance that plunger piston 52 moves up in fluid container 35 during dose setting. As the dose set is selectable by a user, the ready to inject position of the actuator 34 relative to the housing 22 is different for different doses, which provides a visible cue for the user. When the actuator 34 is plunged toward the housing 22, plunger shaft 67 and therefore piston 52 are driven distally a corresponding amount to force the set dose through the injection needle 25, which distal movement of the rack 66. via its relation to gear 58, causes the display to simultaneously dial down toward zero while continuously displaying the amount of medication still remaining to be injected. In order to prevent the plunger shaft 73 and its piston from backing up during injection so as to increase the storage chamber volume, plunging of actuator 34 activates a not shown decoupling clutch within the housing 22 which disengages the drive assembly so as to not drivingly shift the plunger shaft 73 and piston 44 proximally, as well as which preferably locks the storage chamber plunger, such as by introducing a pawl into rack 72 or locking a drive gear, to prevent proximal floating of shaft 73 and piston 44. Such a disengagement may involve axially shifting spur gear 60 to disengage it from gear 58, or vice versa.

Although actuator 34 is described as providing a direct actuation of the injection plunger, it is within the scope of the invention for the actuator operation to involve a mechanical advantage, such as if, for example, the actuator travel length is desired to be increased to be more recognizable. Still further the actuator can serve as a trigger that initiates a spring assisted, or possibly fully automatic, advancement of the injection plunger to expel the set dose from the injection chamber.

Because the pistons 44, 52 may translate without rotation during use such as in the shown embodiment, cross-sectional piston shapes and chamber shapes different from the circular one shown and described may be employed in alternate embodiments. These other shapes may be used to create fluid containers that, for example, maximize the use of available space to create optimally sized and shaped devices. For example, a dual chamber fluid container having a circular cross-section in overall shape may be provided, but which container has a cross-sectional crescent shaped injection chamber and associated piston, and a storage chamber and associated piston that cross-sectionally is the remainder of the circle. Still further, one of the pistons may be annular, such as if one chamber surrounds the other chamber.

Still further, while the plunger and drive assembly configuration shown in Fig. 2 is suitable, other designs may be substituted within the scope of the invention. For example, the plunger shafts may rotate during advancement, such as if the chamber sealing pistons were rotatably mounted thereto. In another embodiment, the gearing pair 58 and 60 could be replaced with a single gear that meshes with different diameter pinion portions of the worm gears. However, such gear pair 58 and 60 advantageously allows the exact same sized and shaped part to be used as spur gear 62/worm gear 64 and spur gear 68/worm gear 70, as gear pair 58 and 60 serve to alone provide the six to one ratio described above, or other gear ratio desired by the manufacturer. Still further, and rather than spur gears, bevel gears may be used to transmit driving forces. And, one of the bevel gears may be spring loaded to an engaged position, which gear can be a part of the decoupling clutch activated by actuator plunging. When the actuator plunging occurs, the spring loaded gear is shifted against the force of its returning spring to a disengaged state, which state results in the storage chamber plunger not being driven distally.

The structure of injector pen 20 will further be understood in view of the following brief explanation of its general operation. With injector pen 20 arranged as shown in Fig. 1, when a user needs to select and inject a dose of the medicine, the user rotates wheel 30 in a first direction to dial up display 33 to indicate the particular dose desired to be injected at that time. As wheel 30 is being rotated to increase the dose setting, plunger shaft 67 and its associated piston 52 are shifted upward within tubular portion 39, while plunger shaft 73 and its associated piston 44 are shifted downward within tubular portion 37 one-sixth the upward travel distance of piston 52, thereby generally maintaining the equistatic pressure of the medication within the fluid container reservoir. As plunger shaft 67 and piston 52 shift upward, actuator 34 simultaneously shifts away from housing 22 an identical amount. If the user causes display 33 to show more than the dose desired to be delivered, wheel 30 can be rotated in the opposite direction, which causes the plunger shafts and pistons to move in the opposite direction as during dose increasing, as well as causes actuator 34 to move closer to the housing 22. Furthermore, the pen may also be dialed back to the zero position if it is not desired to use the medication at that time.

After the user has set injector pen 20 to deliver the desired dose, the user merely maneuvers the injector pen such that the tip 26 of injection needle 25 penetrates the injection site, and then manually plunges actuator 34 toward housing 22. Plunging of actuator 34 causes plunger movement that reduces the injection chamber volume while not altering the storage chamber volume, thereby resulting in the medication previously within injection chamber 50 being forced through needle 25 into the injection site. An end of injection click may be provided, such as via the use of a detent or the like, to provide an audible indication to a user of when the injection is complete.

Pen 20 can continue to be used to deliver medicine until not enough medicine remains in the pen to deliver a desired dose, which can be indicated to a user in a variety of ways. For example, the user may be unable to dial up such dose because during dose setting the plunger piston 44 bottoms out at wall 46. at which time the dial would indicate the limited amount still available to deliver. When insufficient medicine remains, pen 20 is to be disposed of, typically after administering the amount remaining with the expectation of delivering the shortfall with the next pen, and replaced with a similar but entirely new pen.

The multiple chamber injector pen of the present invention can be adapted to serve as a reusable injector pen. In such an embodiment, after the quantity of medicine contained in its fluid container is exhausted by multiple operations of the pen, the container will be removed, the plungers will be reset, and a replacement fluid container will be loaded into the pen for subsequent use.

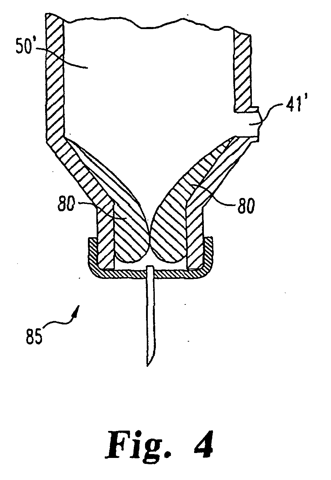

Referring now to Fig. 4, there is diagrammatically shown in cross-section a portion of the injection chamber of another pen of the present invention. To accommodate minor hydraulic pressure variance in the reservoir, possibly due to manufacturing tolerances in the multiple plunger design or tolerance stack up in the gear set, a conical one-way valve 80 is positioned in the distal region of injection chamber 50' that ports to the storage chamber via channel 41'. Valve 80 is elastomeric and can contract and expand small amounts without losing its seal during plunger movements associated with dose setting. In the event that the plungers do not move precisely as designed, and as-built the storage chamber plunger moves down slightly more than intended during dose setting, rather than medicine being ejected from the needle, valve 80 expands slightly downward to account for the additional volume forced into the injection chamber while maintaining adequate dose accuracy. Conversely, in the event that as-built the storage chamber plunger moves down slightly less than intended during dose setting, rather than air being drawn in through the needle, valve 80 contracts slightly upward to account for the lesser volume forced into the injection chamber while maintaining adequate dose accuracy. Only when the injection chamber piston is forcefully thrust down during injecting is the medicine pressurized sufficiently to open the one way valve 80 to allow medicine to pass and enter the proximal tip of the injection needle which, when the pen needle assembly 85 is mounted to the housing as shown, does not penetrate valve 80 but rather is located between the valve and the distal end of the fluid container. The distal end may be provided with an additional, not shown septum

One possible valve for such a design is known as the MicroBarrier valve by Waterfall Company, Inc. of Los Altos Hills. California.

In an alternate and not shown embodiment, instead of using a one-way valve to accommodate minor hydraulic pressure variance in the reservoir, the storage chamber piston 44 can be formed to account for such variance. For example, the piston 44 can be formed with a pliable pocket or diaphragm. By having such pocket or diaphragm be able to shift small amounts relative to the sealing body of the piston, pressure changes can be addressed without negatively affecting dose accuracy.

Injection pen 20 is shown as a dual chamber apparatus that delivers a single therapeutic. In alternate embodiments, a three, four or more chamber apparatus can be provided to deliver one of a multiple of, or mixtures of, therapeutics. In such a device, several storage chambers are provided, each capable of housing a different material or therapeutic. The storage chambers are in fluid flow communication with a common injection chamber, which fluid flow connections are preferably provided with one-way valves to ensure no backflow from the injection chamber to the storage chambers. The drive assemblies and plungers are configured such that dose setting can occur separately with respect to each of the storage chambers, but in each case the dosed quantity is introduced into the single injection chamber for subsequent injection via operation of a single actuator.

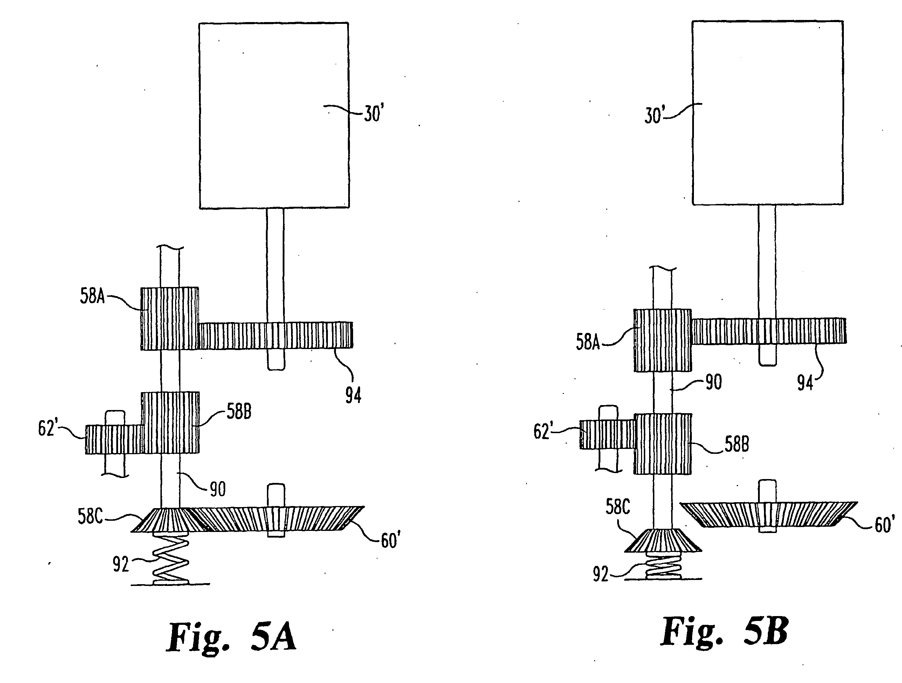

Referring now to Figs. 5A and 5B, there is shown select portions of another embodiment of the medication dispensing apparatus of the present invention, in which embodiment a gear design is further shown which facilitates a suitable decoupling during dose injecting. This decoupling results in the storage chamber plunger not being drivingly plunged, while not preventing the display from simultaneously dialing down toward zero while continuously displaying the amount of medication still remaining to be injected.

As shown in Fig. 5A, which illustrates the portions during dose setting, the apparatus includes a spur gear 58A, a spur gear 58B, and a bevel gear 58C that are each axially and rotatably fixed on a shaft 90 journaled in the housing and axially shiftable therein. A spring 92 acting between the housing and bevel gear 58C biases the shaft and gears 58A-C toward the position shown in Fig. 5A. Spur gear 58A intermeshes with a larger diameter, axially fixed spur gear 94 that is rotatably driven by manual rotation of a dose setting wheel abstractly shown at 30'. It will be appreciated that if it is desired that only the display rotate down during dose injecting, and that the dose setting wheel not rotate, the gear 94 can be connected to the display instead of wheel 30', and the decoupling during injecting of the storage chamber plunger can be adapted to also decouple the rotating display from the dose setting wheel.

Spur gear 58B intermeshes with an axially fixed spur gear 62' that when rotated operates a not shown worm gear and the like similar to the embodiment of Fig. 2 to similarly move a piston within the injection chamber.

Bevel gear 58C intermeshes with an axially fixed bevel gear 60' during dose setting. When gear 60' is rotated by gear 58C, a not shown bevel gear rotatably fixed to a not shown worm gear and the like similar to the embodiment of Fig. 2 operate to move a piston within the storage chamber.

With reference to Fig. 5B, when the not shown actuator is plunged during injecting, shaft 90 and gears 58A-C are shifted axially against the returning force of a now compressed spring 92. Spur gears 58A and 58B, due to their larger axial lengths, remain intermeshed with spur gear 94 and spur gear 62', respectively, to allow rotation transmission. Bevel gear 58C is axially spaced from and does not intermesh with bevel gear 60' such that rotation is not transmitted thereto. When injection is completed and the user stops applying force to the actuator, spring 92 returns shaft 90 and gears 58A-C to the position shown in Fig. 5A.

While this invention has been shown and described as having preferred designs, the present invention may be modified. For example, the apparatus may be a fixed dose pen, such that preparing the pen for delivery involves moving it from a ready to dose position to a predetermined ready to inject position for each actual injection. This application is therefore intended to cover any variations, uses or adaptations of the invention using its general principles.

## Claims

1. A medication dispensing apparatus comprising:
a fluid container defining a medication-filled reservoir including an injection chamber and a storage chamber in fluid flow communication, said fluid container including an outlet;
a first plunger including a piston sealing an end of said injection chamber, said first plunger movable relative to said fluid container while maintaining the seal to alter the volume of said injection chamber;
a second plunger including a piston sealing an end of said storage chamber, said second plunger movable relative to said fluid container while maintaining the seal to alter the volume of said storage chamber;
said sealed end of said injection chamber having a cross-sectional area that is smaller than a cross-sectional area of said sealed end of said storage chamber;
a drive assembly drivingly connected to said first plunger and said second plunger, said drive assembly operable during dose setting to shift said first and second plungers relative to said fluid container different distances so as to reduce the volume of said storage chamber while simultaneously increasing the volume of said injection chamber a similar amount; and
an actuator movable to shift said first plunger relative to said fluid container to reduce the volume of said injection chamber to force medicine in said reservoir through said outlet.

2. The medication dispensing apparatus of claim 1 wherein said second plunger does not shift relative to said fluid container to increase the volume of said storage chamber during movement of said actuator to shift said first plunger to reduce the volume of said injection chamber.

3. The medication dispensing apparatus of claim 1 wherein said outlet is provided by an injection needle that extends through a septum sealing an opening in said fluid container to said injection chamber, whereby said injection needle is in fluid flow communication with said reservoir.

4. The medication dispensing apparatus of claim 1 wherein said fluid container comprises a first tubular portion and a second tubular portion each having an interior hollow, said injection chamber comprising said first tubular portion interior hollow, said storage chamber comprising said second tubular portion interior hollow, each of said first and second tubular portions being arranged laterally of the other.

5. The medication dispensing apparatus of claim 4 wherein said first and second tubular portions are cylindrical and parallel to each other.

6. The medication dispensing apparatus of claim 1 wherein said first plunger comprises a rotatably fixed rack portion, and wherein said drive assembly comprises a worm gear engaged with said rack portion.

7. The medication dispensing apparatus of claim 1 further comprising a dose setting element that operates said drive assembly to shift said first and second plungers relative to said fluid container different distances during dose setting.

8. The medication dispensing apparatus of claim 7 wherein said dose setting element comprises a dial operatively connected to a dose display, and wherein said drive assembly comprises a gear driven by a rotation of said dial, which rotation operates said display during dose setting.

9. The medication dispensing apparatus of claim 1 wherein said drive assembly comprises a plurality of gears.

10. The medication dispensing apparatus of claim 9 wherein said plurality of gears comprise spur gears.

11. The medication dispensing apparatus of claim 9 wherein said first plunger and said second plunger each comprise a rack, wherein said plurality of gears comprises a first worm gear engaged with said first plunger rack and a second worm gear engaged with said second plunger rack, and wherein said first and second worm gears comprise the same pitch and thread direction.

12. The medication dispensing apparatus of claim 1 wherein said piston of said second plunger is designed to accommodate hydraulic pressure variance in said reservoir.

## Patentansprüche

1. Ein Medikationsabgabegerät, umfassend:
einen Flüssigkeitsbehälter, der ein mit einer Medikation gefülltes Reservoir bestimmt, das eine Injektionskammer und eine Aufbewahrungskammer in Fluidverbindung miteinander enthält, wobei der Flüssigkeitsbehälter einen Auslass aufweist;
einen ersten Tauchkolben mit einem Kolben, der ein Ende der Injektionskammer abdichtet und der gegenüber dem Flüssigkeitsbehälter bei Aufrechterhaltung der Abdichtung beweglich ist, um das Volumen der Injektionskammer zu verändern;
einen zweiten Tauchkolben mit einem Kolben, der ein Ende der Aufbewahrungskammer abdichtet und der gegenüber dem Flüssigkeitsbehälter bei Aufrechterhaltung der Abdichtung beweglich ist, um das Volumen der Aufbewahrungskammer zu verändern;
wobei das abgedichtete Ende der Injektionskammer eine Querschnittsfläche hat, die kleiner als die Querschnittsfläche des abgedichteten Endes der Aufbewahrungskammer ist;
eine Antriebsanordnung, die mit dem ersten Tauchkolben und dem zweiten Tauchkolben antriebsmäßig verbunden ist und die bei der Dosiseinstellung betätigbar ist, um die ersten und zweiten Tauchkolben gegenüber dem Flüssigkeitsbehälter um unterschiedliche Distanzen zu verstellen, um das Volumen der Aufbewahrungskammer bei gleichzeitiger Vergrößerung des Volumens der Injektionskammer um eine gleiche Größe zu vermindern; und
ein Betätigungselement, um den ersten Tauchkolben gegenüber dem Flüssigkeitsbehälter zu verstellen, um das Volumen der Injektionskammer zu vermindern, um ein in dem Reservoir enthaltenes Medikament durch den Auslass zu drücken.

2. Das Medikationsabgabegerät nach Anspruch 1, bei dem der zweite Tauchkolben sich gegenüber dem Flüssigkeitsbehälter nicht im Sinne einer Vergrößerung des Volumens der Aufbewahrungskammer verstellt, wenn das Betätigungselement zum Verstellen des ersten Tauchkolbens zwecks Verminderung des Volumens der Injektionskammer bewegt wird.

3. Das Medikationsabgabegerät nach Anspruch 1, bei dem der Auslass von einer Injektionsnadel gebildet ist, die sich durch ein Septum erstreckt, das eine Öffnung in dem Flüssigkeitsbehälter zur Injektionskammer abdichtet, wodurch die Injektionsnadel in Fluidströmungsverbindung mit dem Reservoir ist.

4. Das Medikationsabgabegerät nach Anspruch 1, bei dem der Flüssigkeitsbehälter einen ersten tubusförmigen Abschnitt und einen zweiten tubusförmigen Abschnitt umfasst, die jeweils einen inneren Hohlraum haben, wobei die Injektionskammer den inneren Hohlraum des ersten tubusförmigen Abschnitts und die Aufbewahrungskammer den inneren Hohlraum des zweiten tubusförmigen Abschnitts enthält, wobei die ersten und zweiten tubusförmigen Abschnitte seitlich nebeneinander angeordnet sind.

5. Das Medikationsabgabegerät nach Anspruch 4, bei dem die ersten und zweiten tubusförmigen Abschnitte zylindrisch und einander parallel sind.

6. Das Medikationsabgabegerät nach Anspruch 1, bei dem der erste Tauchkolben eine gegen Drehung gesicherte Zahnstange umfasst und die Antriebsanordnung ein Schneckenrad umfasst, das in die Zahnstange eingreift.

7. Das Medikationsabgabegerät nach Anspruch 1, weiterhin enthaltend ein Dosiseinstellelement, das auf die Antriebsanordnung einwirkt, um bei der Dosiseinstellung die ersten und zweiten Tauchkolben gegenüber dem Flüssigkeitsbehälter um unterschiedliche Distanzen zu verstellen.

8. Das Medikationsabgabegerät nach Anspruch 7, bei dem das Dosiseinstellelement ein Einstellrad umfasst, das mit einer Dosisanzeigeeinrichtung wirkungsverbunden ist, und bei dem die Antriebsanordnung ein Zahnrad umfasst, das durch Drehen des Einstellrades angetrieben ist, welches Drehen die Anzeigeeinrichtung bei der Dosiseinstellung betätigt.

9. Das Medikationsabgabegerät nach Anspruch 1, bei dem die Antriebsanordnung mehrere Zahnräder umfasst.

10. Das Medikationsabgabegerät nach Anspruch 9, bei dem die Zahnräder Stirnzahnräder sind.

11. Das Medikationsabgabegerät nach Anspruch 9, bei dem der erste Tauchkolben und der zweite Tauchkolben jeweils eine Zahnstange umfassen, wobei die Zahnräder ein erstes Schneckenrad, das in die erste Zahnstange eingreift, und ein zweites Schneckenrad, das in die zweite Zahnstange eingreift, umfassen und die ersten und zweiten Schneckenräder die gleich Teilung und Steigungsrichtung haben.

12. Das Medikationsabgabegerät nach Anspruch 1, bei dem der Kolben des zweiten Tauchkolbens so gestaltet ist, dass er hydraulische Druckänderungen in dem Reservoir aufnimmt.

## Revendications

1. Appareil de distribution de médicament comprenant:
un contenant pour fluide définissant un réservoir rempli de médicament incluant une chambre d'injection et une chambre de stockage en communication fluidique, ledit contenant pour fluide incluant un orifice de sortie ;
un premier plongeur incluant un piston fermant de manière étanche une extrémité de ladite chambre d'injection, ledit premier plongeur pouvant se déplacer par rapport audit contenant pour fluide tout en maintenant l'étanchéité de manière à modifier le volume de ladite chambre d'injection :
un second plongeur incluant un piston fermant de manière étanche une extrémité de ladite chambre de stockage, ledit second plongeur pouvant se déplacer par rapport audit contenant pour fluide tout en maintenant l'étanchéité de manière à modifier le volume de ladite chambre de stockage ;
ladite extrémité fermée de manière étanche de ladite chambre d'injection ayant une surface de section transversale qui est inférieure à une surface de section transversale de ladite extrémité fermée de manière étanche de ladite chambre de stockage ;
un ensemble d'entraînement raccordé de manière à pouvoir entraîner ledit premier plongeur et ledit second plongeur, ledit ensemble d'entraînement pouvant être mis en action durant le réglage d'une dose de manière à décaler lesdits premier et second plongeurs par rapport audit contenant pour fluide sur des longueurs différentes de façon à réduire le volume de ladite chambre de stockage tout en augmentant simultanément le volume de ladite chambre d'injection d'une quantité similaire ; et
un actionneur pouvant se déplacer de manière à décaler ledit premier plongeur par rapport audit contenant pour fluide de façon à réduire le volume de ladite chambre d'injection de sorte que le médicament dans ledit réservoir est poussé à travers ledit orifice de sortie.

2. Appareil de distribution de médicament selon la revendication 1, dans lequel ledit second plongeur ne se décale pas par rapport audit contenant pour fluide de manière à augmenter le volume de ladite chambre de stockage lors des mouvements dudit actionneur, pour décaler ledit premier plongeur de manière à réduire le volume de ladite chambre d'injection.

3. Appareil de distribution de médicament selon la revendication 1, dans lequel ledit orifice de sortie est équipé d'une aiguille d'injection qui se prolonge au travers d'une cloison fermant de manière étanche un orifice entre ledit contenant pour fluide et ladite chambre d'injection, suite à quoi ladite aiguille d'injection est en communication fluidique avec ledit réservoir.

4. Appareil de distribution de médicament selon la revendication 1, dans lequel ledit contenant pour fluide comprend une première portion tubulaire et une seconde portion tubulaire ayant chacune une cavité intérieure, ladite chambre d'injection comprenant ladite cavité intérieure de la première portion tubulaire, ladite chambre de stockage comprenant ladite cavité intérieure de la seconde portion tubulaire, chacune desdites première et seconde portions tubulaires étant disposées latéralement l'une par rapport à l'autre.

5. Appareil de distribution de médicament selon la revendication 4, dans lequel lesdites première et seconde portions tubulaires sont cylindriques et parallèles l'une à l'autre.

6. Appareil de distribution de médicament selon la revendication 1, dans lequel ledit premier plongeur comprend une portion de support fixée de manière rotative, et dans lequel ledit ensemble d'entraînement comprend une vis sans fin engagée dans ladite portion de support.

7. Appareil de distribution de médicament selon la revendication 1, comprenant en outre un élément de réglage d'une dose qui met en action ledit ensemble d'entraînement pour décaler lesdits premier et second plongeurs par rapport audit contenant pour fluide sur des longueurs différentes au cours du réglage d'une dose.

8. Appareil de distribution de médicament selon la revendication 7, dans lequel ledit élément de réglage d'une dose comprend un bouton tournant connecté de manière fonctionnelle à un dispositif d'affichage de doses, et dans lequel ledit ensemble d'entraînement comprend une engrenage entraîné par une rotation dudit cadran, laquelle rotation met en action ledit dispositif d'affichage au cours du réglage d'une dose.

9. Appareil de distribution de médicament selon la revendication 1, dans lequel ledit ensemble d'entraînement comprend une pluralité d'engrenages.

10. Appareil de distribution de médicament selon la revendication 9, dans lequel ladite pluralité d'engrenages comprend des engrenages à denture droite.

11. Appareil de distribution de médicament selon la revendication 9, dans lequel ledit premier plongeur et ledit second plongeur comprennent chacun un support, dans lequel ladite pluralité d'engrenages comprend une première vis sans fin engagée dans ledit premier support de plongeur et une seconde vis sans fin engagée dans ledit second support de plongeur, et dans lequel lesdites premières et seconde vis sans fin comprennent le même pas et la même direction de filetage.

12. Appareil de distribution de médicament selon la revendication 1, dans lequel ledit piston dudit second plongeur est conçu de manière à s'adapter à la variation de pression hydraulique dans ledit réservoir.
